# EUROPEAN PATENT APPLICATION

(11) **EP 4 748 942 A1**
(43) Date of publication of application: **27.05.2026**
(21) Application number: 24214722.1
(22) Date of filing: 22.11.2024
(51) Int. Cl.: C12Q 1/6806, C12Q 1/6834, C12Q 1/6841

(54) **SPATIAL TRANSCRIPTOME DETECTION IN TISSUE USING ROLONY TAGGING**

(71) Applicant: Miltenyi Biotec B.V. & Co. KG, 51429 Bergisch Gladbach (DE)
(72) Inventor: MEYER, Hansueli, 51429 Bergisch Gladbach (DE); PINARD, Robert, 51429 Bergisch Gladbach (DE); HOSONO, Seiyu, 51429 Bergisch Gladbach (DE); MULLER, Reto, 51429 Bergisch Gladbach (DE)
(74) Representative: Kisters, Michael Marcus

(57) **Abstract**

This invention is about detecting transcript (mRNA) spatially in tissue. The method described allows to significantly increase the detected transcript density.

## Description

The invention is directed to a method for obtaining the sequence information and the spatial information of at least one RNA strand on a sample immobilized on a surface.

### BACKGROUND

Several methods for spatial (non-targeted) transcriptome detection are known, for example as disclosed in US 20140066318, US10030261, US 10774374 or US20210292748

The currently existing methods for spatial transcriptome detection are all based on a concept using a glass surface, array, bead array or substrate where oligonucleotides are immobilized and used to capture mRNA expressed in prepared tissue slices when brought in contact with the glass surface, array or substrate.

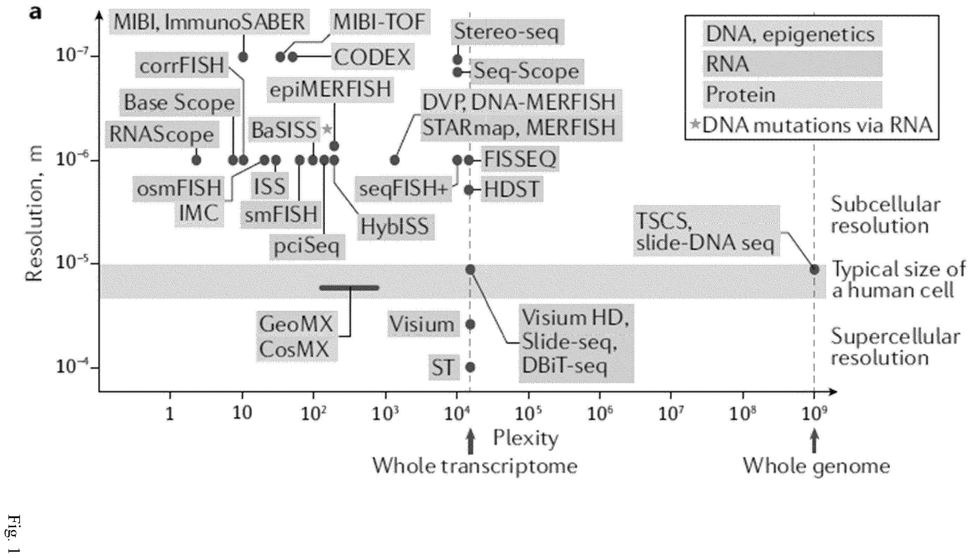

The known methods have some limits in resolution and length of the genes to be sequenced. Fig. 1 shows an overview on the known methods in view of their capabilities (taken from "Spatial biology of cancer evolution", Seferbekova, Nature Sept 2022).

One of the current approaches is to directly target mRNA transcripts in cells with padlock probes and then performing rolling circle amplification to create nanoballs ("rolonies"). Those rolonies are typically in the size of 600-800 nm in diameter. Only one mRNA transcript can be detected per rolony. So in total approximately 50-100 mRNA transcripts can be detected within a cell. The method described here will allow to significantly increase the number of spatially detectable mRNA transcripts per cell and lead to a total of 2500 to 5600.

It was found that mRNAs can be captured and molecularly characterized by providing circular oligomers comprising certain units as disclosed later having the complementary sequences what is actually needed and multiplying the circular oligomers by RCA (rolling circle amplification), thereby obtaining rolonies of concatemers having the "correct" sequences.

### OBJECT OF THE INVENTION

Obj ect of the invention was a method for obtaining the sequence information and the spatial information of at least one RNA strand on a sample immobilized on a surface comprising the steps
a. providing a circular oligonucleotide having 50 - 1000 nucleotides, comprising a complementary cleavable unit having 5-50 nucleotides, a poly-A unit having 5 - 350 adenosine nucleotides, a complementary unique molecular identifier unit having 5 - 50 nucleotides and a complementary sequencing primer unit having 5 - 50 nucleotides
b. multiplying the circular oligonucleotide by a polymerase capable of rolling circle amplification into a plurality of concatemers comprising a cleavable unit, a poly-T unit, a unique molecular identifier (UMI) unit and a sequencing primer unit thereby obtaining rolonies
c. providing the rolonies to the sample, thereby hybridizing the rolonies to at least one RNA strand via the poly-T-units of the concatemers
d. enzymatically cleaving the rolonies at the cleavable units of the concatemers, thereby generating linear oligonucleotides hybridized to at least one RNA strand
e. extending the hybridized linear oligonucleotide using the mRNA as template, thereby forming at least one c-DNA strand
f. sequencing-by-synthesis of the hybridized linear oligonucleotide in 3' direction, starting from the sequencing primer unit, thereby simultaneously obtaining the sequence information of the unique molecular identifier unit and detecting the spatial location of the hybridized linear oligonucleotide
g. removing the c-DNA from the sample
h. sequencing the c-DNA, thereby obtaining the sequence information of the at least one RNA strand
i. aligning the sequence information and the spatial information of the unique molecular identifier unit obtained in step f) with the sequence information of the at least one RNA strand obtained in step h).

Since the circular oligonucleotide is multiplied by rolling circle amplification in step b), the obtained concatemers comprise the complementary sequences of the starting material. In the following, it is referred to the "final" sequences of the cleavable unit, unique molecular identifier and sequencing primer unit, with the consequence that the respective "starting" sequences are referred to as "complementary" sequences i.e. "complementary cleavable unit", "complementary unique molecular identifier" and "complementary sequencing primer unit".

### BRIEF DESCRIPTION OF THE DRAWINGS

Fig. 1. shows a range of spatially resolved approaches for mapping DNA, RNA and proteins has been developed. Higher spatial resolution typically implies smaller molecular quantities. Source: Zaira Seferbekova, Spatial biology of cancer evolution, Nature Review Genetics Sept 2022.

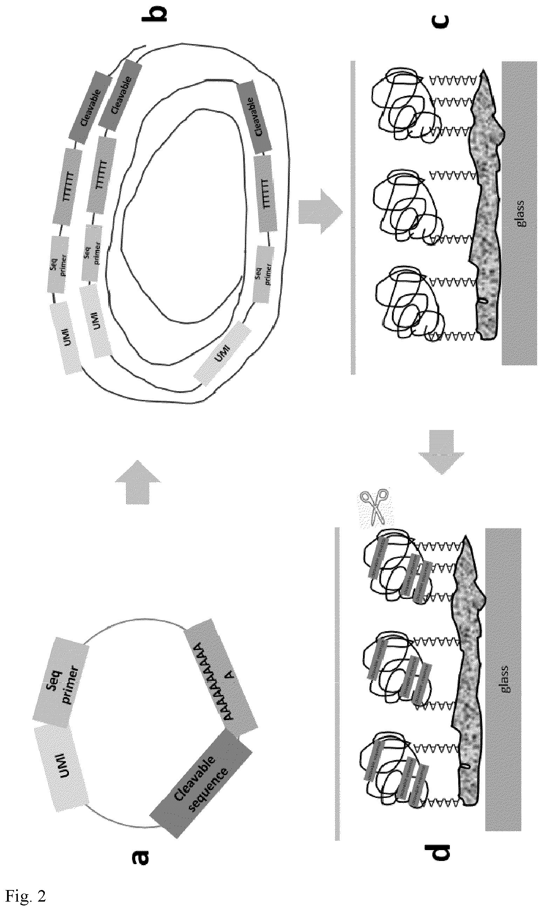

Fig 2. shows a sketch of the steps a) to d) of the method of the invention

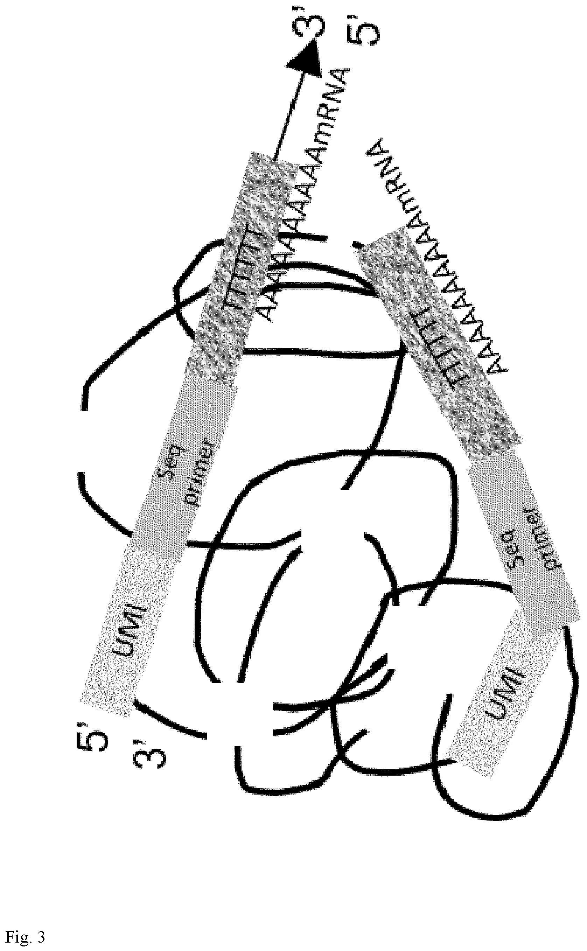

Fig. 3 shows a sketch of step e) of the method of the invention

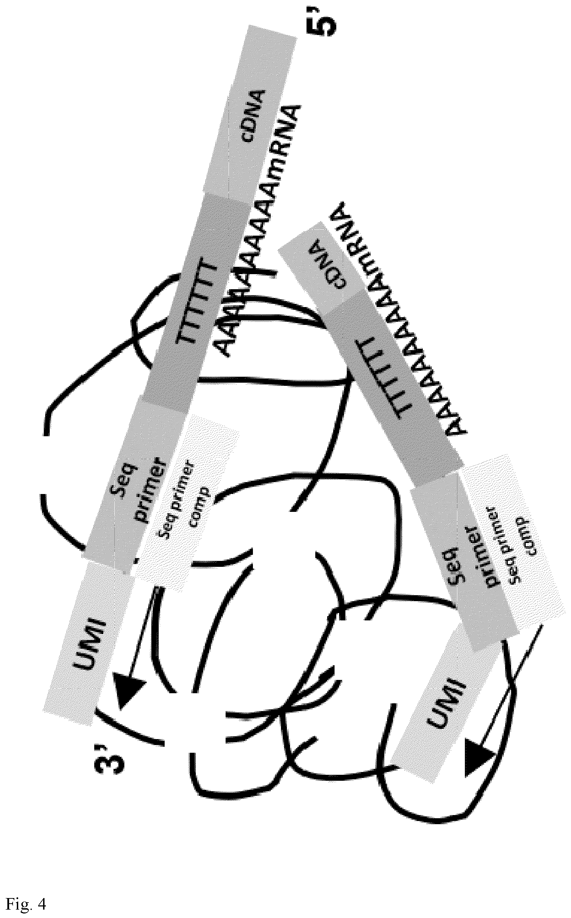

Fig. 4 shows a sketch of a part of step f) of the method of the invention

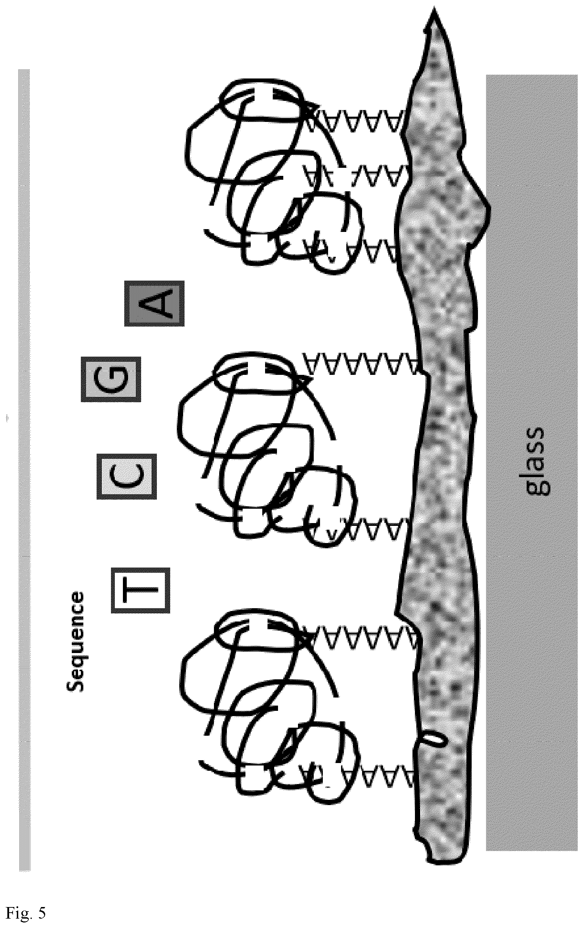

Fig. 5 shows the sequencing by synthesis part of step f) of the method of the invention
Fig. 6 and 7 show images of experimental results obtained with the method of the invention.

### DETAILED DESCRIPTION

One of the current approaches is to directly target mRNA transcripts in cells with padlock probes and then performing rolling circle amplification to create nanoballs ("rolonies"). Those rolonies are typically in the size of 600-800 nm in diameter. Only one mRNA transcript can be detected per rolony. So in total approximately 50-100 mRNA transcripts can be detected within a cell. The method described here will allow to significantly increase the number of spatially detectable mRNA transcripts per cell and lead to a total of 2500 to 5600.

The present invention is directed to a method for obtaining the sequence information and the spatial information of at least one RNA strand on a tissue sample immobilized on a surface comprising the steps providing a circular oligonucleotide containing a cleavable unit, a poly-A unit, a unique molecular identifier (UMI) unit, and a sequencing primer unit.

Preferable, the cleavable units comprise recognition sequences for a restriction enzyme selected from the group consisting of EcoRI, EcoRV, BamHI, HindIII, Notl and PSstl; like for example the recognition sequence 5'-GAATTC-3'.

This oligonucleotide is amplified by rolling circle amplification (RCA) to form concatemers, called rolonies, which are hybridized to an RNA strand via the poly-T units directly on the tissue sample. The rolonies are then enzymatically cleaved at the cleavable units, creating linear oligonucleotides that are extended using the RNA as a template to form c-DNA.

Enzymatically cleaving the rolonies at the cleavable units in step d can be performed by hybridizing an oligonucleotide to the complementary units of the cleavable units and then cleave by using a restriction enzyme.

Sequencing-by-synthesis is performed directly on the tissue sample (in situ sequencing) to obtain sequence information from the UMI strand. Additional steps include removing the c-DNA, sequencing the c-DNA and UMI again, and matching the cDNA sequence with the UMI determined directly in the tissue. This leads to the detection of the transcript and exact location on the tissue.

The surface is preferable a transparent surface and the sequencing-by-synthesis of the hybridized linear oligonucleotide in step f) is preferable performed by detecting fluorescence radiation through the transparent surface.

Further, enzymatically cleaving the rolonies at the cleavable units in step d can be performed by hybridizing an oligonucleotide to the units of the cleavable units and then cleave by using a restriction enzyme.

Sequencing by synthesis is a method to determine the sequence of single stranded DNA we claim sequencing of cDNA reversely transcribed from mRNA.

A polymerase and nucleotides are used to replicate the bases (A,C,G,T) of single stranded DNA. Each nucleotide is modified and contains a blocker group and a fluorescence dye.

Each base (A,C,G,T) has its own fluorescence dye and can therefore be uniquely identified. The modified nucleotides are incorporated in each sequencing cycle. The blocking group prevents that more than one nucleotide is incorporated in one cycle.

The single stranded DNA is extended in multiple cycles to determine the unknown bases. Each cycle contains an extension step, an imaging step to determine the fluorescence color of each incorporated base and a cleave step to remove the blocking group.

In a variant of the method, after removing the c-DNA strand from the sample, the c-DNA strand is multiplied by RCA before sequencing.

Preferable, the c-DNA strand is multiplied by RCA by circularizing the c-DNA with a ligase enzyme (like T4) and using the circularized cDNA as template for RCA.

The blocking group prevents the incorporation of two bases in one cycle.

Sequencing by synthesis is usually carried out in micro fluidic channels where the polymerase, nucleotides and other chemistry components are provided. In many cases the micro fluidic channel is also temperature controlled and higher temperatures are beneficial for nucleotide incorporation (e.g. 65 DC).

The single stranded DNA may be immobilized on glass substrates where the sequencing takes place. In many cases the single stranded DNA is amplified before sequencing takes place and the target strand is replicated many hundred times (bridge amplification, rolling circle amplification, other).

Identifying the spatial location of the unique molecular identifier units in step f) may be achieved/performed by imaging the immobilized tissue sample in each cycle of the sequencing-by-synthesis process.

This allows much higher fluorescence signals to be detected and to be better distinguished from background signals.

As mentioned sequencing by synthesis usually takes place on single stranded DNA that is immobilized on glass substrate. Preferable, the sample is immobilized on the transparent surface by APTES, APTEM, silane, polymer based, glycol, or thiol coating.

In a variant, the rolonies may also be crosslinked on the sample, for example via UTP using crosslinking agents such as bis-PEG

The method described in this application is applying sequencing by synthesis directly on single stranded DNA that is immobilized on cells or a tissue surface.

In this particular case sequencing is being used to decode molecular unique identifier part of a oligomer which is part of a rolony. The rolony was generated by RCA.

This unique molecular identifier allows to uniquely identify the rolony and also provides spatial coordinates in x and y where in the tissue the sequence comes from.

Identifying the spatial location of the unique molecular identifier units in step f) can be performed by imaging the immobilized tissue sample in each cycle of the sequencing-by-synthesis process.

The method of the invention may comprise one or more of the steps outlined in the following schematic workflow:
1. Start with linear oligomers. Each oligomer is buildup of synthesized oligonucleotides with the following sections:
   a. Poly A homopolymer oligos 10-300 bp
   b. Sequencing primer oligos 5-50 bp
   c. Cleavable oligo sequence 5-50 bp
   d. Unique molecular identifier (UMI) oligos 5-50 bp
   e. Additional nucleotides necessary to form the padlock
2. Each individual oligomer further on called padlock with its UMI is circularized. Forming a closed circle and which is still a single stranded oligo. Afterwards a special polymerase is used to start copying the circularized oligo ending up in 300-500 copies of the original oligo sequence. Those copies are also called concatemers. There are millions of rolonys generated in one tube in parallel. Each of those rolonies will have its own unique molecular identifier.
3. Generated rolonies will afterwards be brought in contact with a prepared tissue sample. The tissue sample can be a slice of the tumor tissue biopsy and is usually between 5 and 20 um thick. The tissue itself underwent multiple sample preparation steps.
4. Hybridization of each rolony to the poly A tail of each expressed mRNA and cross linking rolony to the tissue. In addition the poly T moiety in the rolonies generated from the poly A on the padlock is hybridized to the tail of mRNA.
5. Enzymatically cut each rolony into each concatemer (cleavable sequence) so that it generates a 3' end to serve as a primer for reverse transcription reaction performed directly on tissue. Each concatemer will have a UMI and a captured expressed mRNA attached to it.
6. cDNA synthesis of all captured mRNA strands with UMI containing fragmented rolonies at the cleavable sequence.
7. The entire tissue is imaged with a pre staining of DAPI. A sequencing primer is added to each rolony and then the unique molecular identifier (UMI) is sequenced in every rolony. The x and y coordinate of each rolony is recorded.
8. Extract DNA from the tissue slide, which contains the reverse transcribed cDNA strand with a specific UMI. Further downstream library prep. Rolony making again of each concatemer and then ex situ sequencing. The UMI is sequenced again and also some of the target region of the captured mRNA. Afterwards the x y location of where the mRNA came from in the tissue can be matched with the UMI determined in step 7.

### EXAMPLES

Experiments were carried out to test the approach described in this application. The experiment is simplified where the oligomers being used are only containing a very short unique molecular identifier and a sequencing primer region. Target tissue type for the present application is formalin-fixed and paraffin embedded (FFPE) tissue. Therefore the described steps start with a FFPE tissue from a tissue block. In the particular case a FFPE tonsil block.

### Preparing the tissue and slicing on microtome and adding to transparent glass slide

The tissue block, embedded in a paraffin wax casing, is trimmed on the microtome to remove excess paraffin and expose the tissue surface. This process, known as "facing," creates a smooth, even surface. To make slicing easier and improve section quality, the tissue block is often chilled in the case here frozen at -20 degree Celsius. The block is clamped into the microtome's chuck, holding it in a stable position to prevent movement during slicing. The desired thickness for each slice, typically between 4-10 micrometers (µm), is set on the microtome.

The microtome's mechanism gradually advances the block towards a sharp steel or disposable blade. As the block advances, the blade slices through the tissue, creating thin, ribbon-like sections. The ribbon is placed on a warm water bath, which helps flatten the tissue sections by gently relaxing the paraffin. Individual sections are lifted from the water bath using glass slides, typically with the tissue side facing up. The glass slides are then dried and stored at 4 degrees Celsius until usage.

### Further prepare the transparent glass slide with the tissue

The glass slide with the tissue slice on it is usually placed in xylene or similar solvent to dissolve and remove the paraffin (wax). Afterwards the tissue is rehydrated though a graded ethanol series (e.g. 95%, 90%, 70%, 50%). And finally rinsed with water to remove any residuals.

Another important step for tissue preparation is to break crosslinks formed during fixation. The tissue is heated in buffer solution at a controlled pH. This can be a professional pressure cooker or industrial steamer (water temperature is 95 up to 99 degrees Celsius).

The tissue might be further RNA stabilized with RNase inhibitor. Generating rolonies on the tissue to detect mRNA

In the present experiment four different mRNA were targeted to directly detect in tissue. Therefore the oligomers being used containing a very short unique molecular identifier and a sequencing primer region plus the oligos of the arms of the padlock probe are designed to bind to the target mRNA on the target gene. When the probe encounters its mRNA target, the two ends hybridize in a "lock-and-key" fashion.

After hybridization a DNA ligase enzyme is used to ligate the two arm and forming a circular probe. After the ligation the circularized probe serve as template for rolling circle amplification (RCA). With that the tissue is ready to undergo sequencing by synthesis step after adding a sequencing primer to each rolony.

### Scanning the entire tissue to get morphology and location of each cell

Before doing sequencing by synthesis the tissue is fully imaged with a fluorescence microscope.

The cells or tissue can be stained with DAPI (4',6-diamidino-2-phenylindole) to visualize the cell nucleus or an antibody marker to stain the cell membrane.

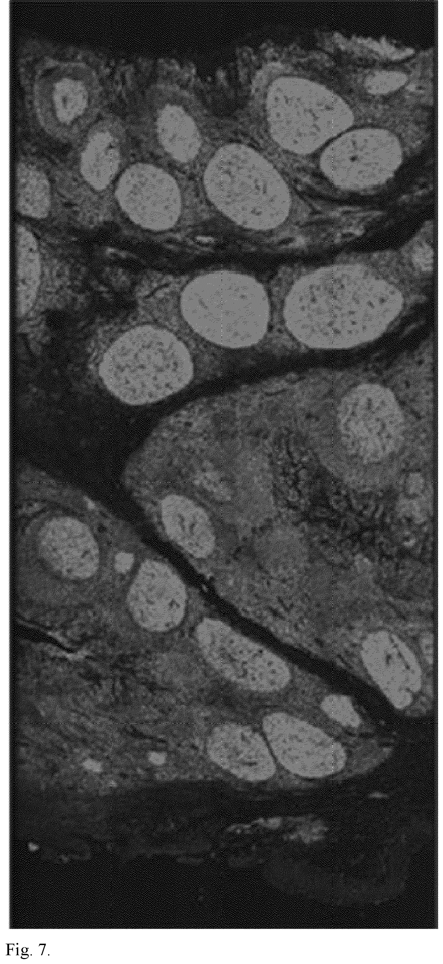

The picture in Fig. 7. shows a fully imaged tonsil tissue slice 3 x 7 mm in size with DAPI stained nucleus with a fluorescence microscope. The cells or tissue can be stained with DAPI (4',6-diamidino-2-phenylindole) to visualize the cell nucleus or an antibody marker to stain the cell membrane.

After the entire tissue is imaged sequencing by synthesis can take place to determine the sequence of the single stranded DNA.

### Performing sequencing by synthesis to identify the UMI and spatial location of the mRNA in tissue

The sequencing by synthesis process uses fluorescently labeled nucleotides. DNA polymerase sequentially incorporates each labeled nucleotide into the DNA strand based on the template, and the specific fluorescent signal is recorded after each cycle.

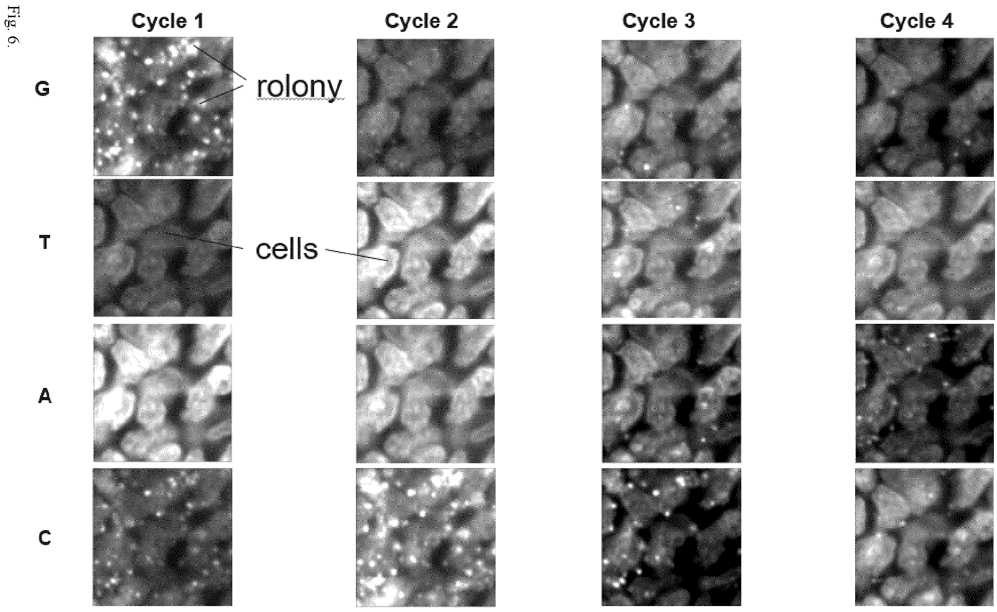

Fig 6 shows sequencing by synthesis on tissue for multiple cycles showing rolonies lighting up in different bases A, C, G or T.

As indicated on the picture the stained cells are directly visible in each fluorescence channel. Rolonies are lighting up in the different channels when applying the sequencing by synthesis chemistry in each cycle.

An image software algorithm overlays each image for G, T, A and C channel in each cycle and determines the sequence of each rolony (for example "GCTAGCTT,..). The rolonies are lighting up either in G, T, A or C channel depending on the cycle. The sequence and spatial coordinates in x and y are recorded. So each unique molecular identifiers will be identified and the location on the tissue will be known.

This is an example on how the UMI code can be determined for the spatial transcriptome detection in tissue using rolony tagging.

The sequence is then equal to the UMI of this particular rolony and can be spatially located together with the tissue image.

## Claims

1. Method for obtaining the sequence information and the spatial information of at least one RNA strand on a sample immobilized on a surface comprising the steps
a. providing a circular oligonucleotide having 50 - 1000 nucleotides, comprising a complementary cleavable unit having 5-50 nucleotides, a poly-A unit having 5-350 adenosine nucleotides, a complementary unique molecular identifier unit having 5 - 50 nucleotides and a complementary sequencing primer unit having 5 - 50 nucleotides
b. multiplying the circular oligonucleotide by a polymerase capable of rolling circle amplification into a plurality of concatemers comprising a cleavable unit, a poly-T unit, a unique molecular identifier (UMI) unit and a sequencing primer unit thereby obtaining rolonies
c. providing the rolonies to the sample, thereby hybridizing the rolonies to at least one RNA strand via the poly-T-units of the concatemers
d. enzymatically cleaving the rolonies at the cleavable units of the concatemers, thereby generating linear oligonucleotides hybridized to at least one RNA strand
e. extending the hybridized linear oligonucleotide using the mRNA as template, thereby forming at least one c-DNA strand
f. sequencing-by-synthesis of the hybridized linear oligonucleotide in 3' direction, starting from the sequencing primer unit, thereby simultaneously obtaining the sequence information of the unique molecular identifier unit and detecting the spatial location of the hybridized linear oligonucleotide
g. removing the c-DNA from the sample
h. sequencing the c-DNA, thereby obtaining the sequence information of the at least one RNA strand
i. aligning the sequence information and the spatial information of the unique molecular identifier unit obtained in step f) with the sequence information of the at least one RNA strand obtained in step h).

2. Method according to claim 1 **characterized in that** the surface is a transparent and the sequencing-by-synthesis of the hybridized linear oligonucleotide in step f) is performed by detecting fluorescence radiation through the transparent surface.

3. Method according to claim 1 or 2 **characterized in that** the cleavable units comprise recognition sequences for a restriction enzyme selected from the group consisting of EcoRI, EcoRV, BamHI, HindIII, Notl and PSstl.

4. Method according to any of claims 1 to 3 **characterized in that** the cleavable units comprises the recognition sequence 5'-GAATTC-3'.

5. Method according to any of claims 1 to 4 **characterised in that** enzymatically cleaving the rolonies at the cleavable units in step d is performed by hybridizing an oligonucleotide to the cleavable units and then cleave by using a restriction enzyme.

6. Method according to any of claims 1 to 5 **characterised in that** the sequencing-by-synthesis of the unique molecular identifier units in step f) is performed by adding a polymerase capable of incorporating fluorescently labelled and unlabelled nucleotides to determine in a cyclic manner the sequence of the unique molecular identifier units.

7. Method according to any of claims 1 to 6 **characterised in that** identifying the spatial location of the unique molecular identifier units in step f) is performed by imaging the immobilized tissue sample in each cycle of the sequencing-by-synthesis process.

8. Method according to any of claims 1 to 7 **characterised in that** the rolonies are crosslinked on the sample.

9. Method according to any of claims 1 to 8 **characterised in that** the sample is stained before removing of the c-DNA strand from the tissue.

10. Method according to any of claims 1 to 9 **characterised in that** after removing the c-DNA strand from the sample, the c-DNA strand is multiplied by rolling circle amplification before sequencing.

11. Method according to claim 10 **characterised in that** the c-DNA strand is multiplied by rolling circle amplification by circularizing the c-DNA with a ligase enzyme and using the circularized cDNA as template for RCA.

12. Method according to any of claims 1 to 11 **characterised in that** sequencing the c-DNA in step h) is performed by a sequencing-by-synthesis process.

13. Method according to any of claims 1 to 12 **characterised in that** the sample is immobilized on the transparent surface by APTES, APTEM, silane, polymer based, glycol, or thiol coating.
